# DEMANDE DE BREVET EUROPEEN

(11) **EP 1 253 137 A1**
(43) Date de publication de la demande: **30.10.2002**
(21) Numéro de dépôt: 02290905.5
(22) Date de dépôt: 11.04.2002
(51) Int. Cl.: C07C 213/08, C08F 20/34

(54) **Procédé de fabrication du chlorure de l'acrylate de 1,3-bis (dimethylbenzylammonium) isopropyle seul ou en mélange avec d'autres monomères et (co)polymères correspondants**

(30) Priorité: 26.04.2001 FR 0105610
(71) Demandeur: Atofina, 92800 Puteaux (FR)
(72) Inventeur: Riondel, Alain, 57600 Forbach (FR)
(74) Mandataire: Rieux, Michel

(57) **Abrégé**

On prépare un composé de formule (I) seul ou en combinaison avec au moins un monomère de formule (II) : on introduit l'agent quaternisant chlorure de benzyle dans une solution, dans un solvant choisi parmi les composés des formules (I) et (II) et leurs mélanges, du composé de formule (III) à une température de 35 à 60°C, puis on ajoute de l'eau et on laisse se dérouler la réaction jusqu'à conversion complète ou sensiblement complète du composé (III), on sépare une solution aqueuse de composé recherché (I) le cas échéant avec au moins un composé (II) si on a utilisé celui-ci ou ceux-ci comme solvant, et on élimine l'eau le cas échéant.

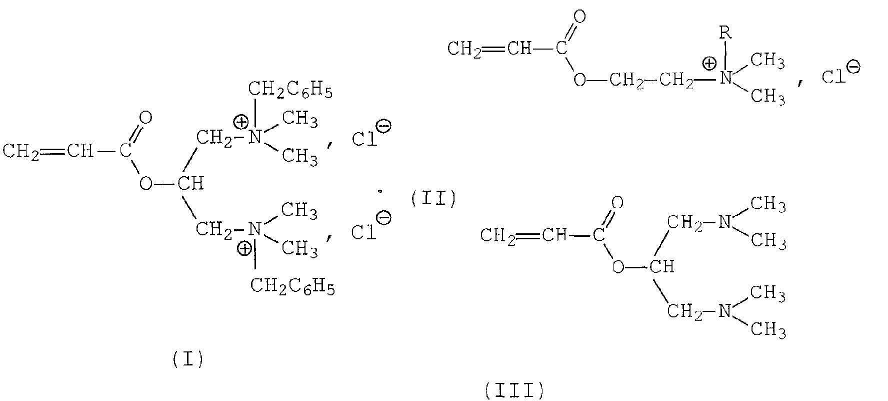

R = -CH₃ ou -CH₂C₆H₅.

## Description

La présente invention concerne le monomère à deux groupes amino quaternaire de formule (I) : 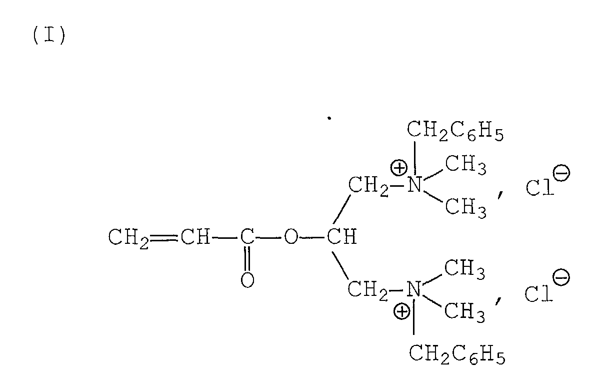 qui est le chlorure de l'acrylate de 1,3-bis (diméthylbenzylammonium) isopropyle (également désigné par l'abréviation S-ADAMQUAT 2BZ).

Par la demande de brevet n° 00-00834 déposée le 24 janvier 2000 et ayant pour titre "Procédé de fabrication de monomères à deux groupes amino quaternaires et les (co)polymères obtenus à partir de ces monomères", il est connu de préparer le monomère de formule (I) en solution aqueuse par le procédé consistant à introduire l'agent quaternisant chlorure de benzyle dans une solution, dans le chloroforme, le dichlorométhane ou le dichloroéthane, de l'acrylate de (2-diméthylamino-1-diméthylaminométhyl)éthyle (également désigné par l'abréviation S-ADAME), à une température de 35 à 80°C, puis on laisse se dérouler la réaction à ladite température jusqu'à disparition complète ou sensiblement complète du S-ADAME, puis on sépare une solution aqueuse de ce composé, l'eau pouvant être éliminée le cas échéant.

Par la demande de brevet français n° 00-00833 déposée le 24 janvier 2000 et ayant pour titre "Dispersions aqueuses sans sel de copolymères hydrosolubles à base de monomères cationiques, leur procédé de fabrication et leurs applications", on connaît une dispersion aqueuse sans sel d'un copolymère hydrosoluble obtenu à partir d'une composition de monomères, comprenant, pour 100 parties en moles :
(a) de 0,5 à 99,5 parties en moles d'au moins un monomère qui peut être le S-ADAMQUAT 2BZ ; et
(b) de 99,5 à 0,5 parties en moles d'au moins un monomère qui peut être choisi, entre autres, parmi le chlorure d'acryloxyéthyltriméthyl ammonium (ADAMQUAT MC) et le chlorure d'acryloxyéthyldiméthylbenzyl ammonium (ADAMQUAT BZ) ;
ladite composition de monomères pouvant renfermer, pour 100 parties en moles de (a) + (b) :
(a) jusqu'à 30 parties en moles d'au moins un monomère hydrophobe ; et/ou
(b) jusqu'à 10 parties en moles d'au moins un monomère réticulant ; et/ou
(c) jusqu'à 30 parties en moles d'au moins un monomère amphiphile.

Ces dispersions aqueuses sans sel contiennent notamment, pour 100 parties en poids :
(A) de 5 à 50 parties en poids de copolymère dispersé à base de la composition des monomères (a) à (e) tels que définis ci-dessus ; et
(B) de 0,5 à 25 parties en poids d'au moins un (co)polymère dispersant,
le complément étant constitué par l'eau.

Elles sont fabriquées par polymérisation radicalaire en milieu aqueux du ou des monomères (a) à (e) tels que définis ci-dessus, en présence du ou des dispersants polymères (B). Et elles sont utiles comme agents de floculation pour le traitement des eaux usées ; agents de rétention de fibres et charges dans les procédés de fabrication du papier ; agents facilitant le nettoyage de supports tels que le textile ; agents de dispersion de charges ; agents d'inhibition pour le transfert de pigments et colorants sur divers supports tels que le textile, épaississants, et agents déshydratants.

Par la demande de brevet français n° 00-00832 déposée le 24 janvier 2000 et ayant pour titre "Dispersions aqueuses salines de (co)polymères hydrosolubles à base de monomères cationiques, leur procédé de fabrication et leurs applications", on connaît une dispersion aqueuse saline d'un (co)polymère hydrosoluble obtenu à partir d'une composition de monomères hydrosolubles, comprenant, pour 100 parties en moles :
(1) de 2 à 100 parties en moles d'au moins un monomère qui peut être le S-ADAMQUAT 2BZ ; et
(2) de 0 à 95 parties en moles d'au moins un monomère qui peut être choisi, entre autres, parmi le chlorure d'acryloxyéthyltriméthyl ammonium (ADAMQUAT MC) et le chlorure d'acryloxyéthyldiméthylbenzyl ammonium (ADAMQUAT BZ),
   d'autres monomères (2) à (5) décrits dans cette demande de brevet français pouvant également être présents, dont le monomère (3) :
(3) de 0 à 95 parties en moles d'au moins un monomère qui peut être choisi, entre autres, parmi le chlorure d'acryloxyéthyltriméthyl ammonium (ADAMQUAT MC) et le chlorure d'acryloxyéthyldiméthylbenzyl ammonium (ADAMQUAT BZ).

Ces dispersions aqueuses salines contiennent notamment, pour 100 parties en poids :
(A) de 10 à 50 parties en poids de (co)polymère dispersé à base de la composition desdits monomères (1) à (5);
(B) de 0,5 à 25 parties en poids d'au moins un (co)polymère dispersant ; et
(C) de 10 à 45 parties en poids d'au moins un sel minéral tel que la solution aqueuse dudit sel dissout le (co)polymère dispersant sans dissoudre le (co)polymère dispersé formé en cours de polymérisation,
le complément étant constitué par l'eau.

Elles sont fabriquées par polymérisation radicalaire en milieu aqueux salin du ou des monomères (1) à (5) en présence dudit ou desdits dispersants polymères (B) et dudit ou desdits sels minéraux (C). Et elles sont utiles comme agents de floculation pour le traitement des eaux usées ; agents déshydratants ; agents de rétention de fibres et charges dans les procédés de fabrication du papier ; agents facilitant le nettoyage de supports tels que le textile ; agents de dispersion de charges ; agents d'inhibition pour le transfert de pigments et colorants sur divers supports tels que le textile ; et épaississants.

La demande de brevet français n° 00-00835 déposée le 24 janvier 2000 et ayant pour titre "Dispersions aqueuses salines de copolymères hydrosolubles à base de monomères cationiques, leur procédé de fabrication et leurs applications" décrit des compositions également à base de S-ADAMQUAT 2BZ, d'ADAMQUAT MC et d'ADAMQUAT BZ.

Or, il apparaît que le procédé actuel de synthèse du S-ADAMQUAT 2BZ n'est pas industrialisable dans des conditions économiques. De plus, comme il implique la présence d'un solvant, les conditions environnementales ne sont pas idéales (problème des VOC).

La Société déposante a donc recherché une solution à ce double problème et elle a découvert que, de façon surprenante, l'utilisation d'au moins l'un parmi l'ADAMQUAT BZ, l'ADAMQUAT MC et le S-ADAMQUAT 2BZ au lieu du chloroforme ou du chlorure de méthylène actuellement utilisés, permettait de réduire la durée de réaction, multipliant ainsi au moins par deux la productivité tout en supprimant la contrainte environnementale liée au solvant.

La présente invention a donc pour objet un procédé de fabrication du composé de formule (I) : 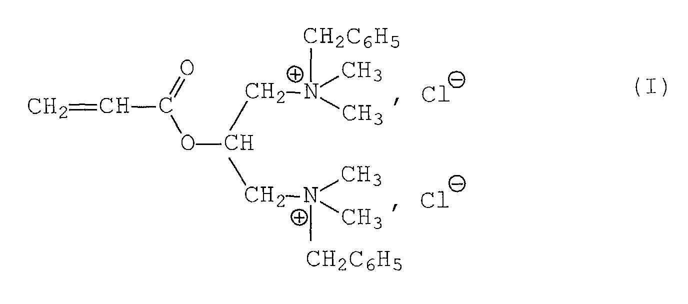 seul ou en combinaison avec au moins un monomère de formule (II) : 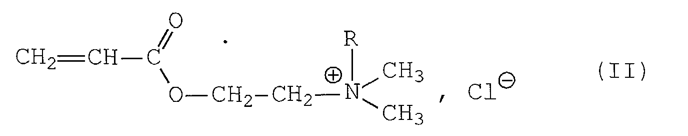 dans laquelle R représente -CH₃ ou -CH₂C₆H₅,
caractérisé par le fait que l'on introduit l'agent quaternisant chlorure de benzyle dans une solution, dans un solvant choisi parmi les composés des formules (I) et (II) et leurs mélanges, du composé de formule (III) : 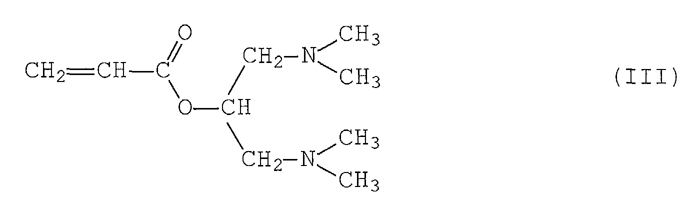 à une température de 35 à 60°C, en particulier de 40 à 50°C, puis qu'on ajoute de l'eau et on laisse se dérouler la réaction jusqu'à conversion complète ou sensiblement complète du composé (III), qu'on sépare une solution aqueuse de composé recherché (I) le cas échéant avec au moins un composé (II) si on a utilisé celui-ci ou ceux-ci comme solvant, et qu'on élimine l'eau le cas échéant.

Avantageusement, on conduit la réaction avec un rapport molaire composé (III)/chlorure de benzyle compris entre 1,6 et 2,0, en particulier entre 1,7 et 1,9. On introduit le chlorure de benzyle dans la solution du composé (III) généralement en l'espace de 8-16 heures, on introduit l'eau dans la solution du composé (III) généralement en l'espace de 2-6 heures, et on commence généralement l'introduction d'eau lorsque 10 à 80% du chlorure de benzyle ont été introduits.

Le procédé est par ailleurs avantageusement conduit en présence d'au moins un stabilisant choisi parmi l'éther méthylique de l'hydroquinone, l'hydroquinone, le 3,5-ditert.-butyl-4-hydroxytoluène et leurs mélanges, à raison notamment de 200 à 2000 ppm par rapport à la solution aqueuse finale et/ou en présence d'au moins un séquestrant de métaux, à raison notamment de 10 à 100 ppm par rapport à la solution aqueuse finale.

Les agents séquestrants pour métaux sont choisis notamment parmi l'acide diéthylène triamine penta acétique, le sel pentasodique de l'acide diéthylène triamine penta acétique, l'acide N-hydroxyéthyl-éthylène diamine triacétique et le sel trisodique de l'acide N-hydroxyéthyl-éthylène diamine triacétique. D'une manière générale, les agents séquestrants sont ajoutés sous la forme d'une solution aqueuse, car ils sont généralement disponibles sous cette forme. Ainsi le sel pentasodique de l'acide diéthylène triamine penta acétique commercialisé sous la dénomination VERSENEX 80 se présente sous la forme d'une solution aqueuse à 40 % en poids environ.

Le procédé selon l'invention peut suivant le cas conduire à une solution aqueuse de composé (I) uniquement, le composé (I) ayant une concentration de 50 à 75 % en poids, ou conduire à une solution aqueuse de composés (I) et (II), la solution aqueuse ayant la composition suivante, pour 100 parties en poids :
- composé (I) : 10 à 70 % en poids
- composé(s) (II): 10 à 70 % en poids
- eau : 20 à 80 % en poids.

Ces solutions peuvent être utilisées pour la polymérisation directement ou en mélange avec d'autres comonomères.

La présente invention a également pour objet des homopolymères ou copolymères comportant des motifs du monomère (I) et le cas échéant d'au moins un monomère (II), ce ou ces monomères ayant été obtenus par le procédé tel que défini ci-dessus.

Ces polymères peuvent être des polymères hydrosolubles ou hydrophobes ayant une présentation sous forme de dispersion aqueuse, latex, solution aqueuse, émulsion inverse ou de poudre. Ils sont préparés par copolymérisation radicalaire selon divers procédés de synthèse tels que les procédés de polymérisation en dispersion, solution, émulsion directe, émulsion inverse et suspension inverse.

L'exemple suivant illustre la présente invention sans toutefois en limiter la portée. Dans cet exemple, les parties et pourcentages indiqués sont en poids sauf indication contraire, et les abréviations suivantes ont été utilisées :

| | |
|---|---|
| S-ADAME | acrylate de (2-diméthylamino-1-diméthylaminométhyl) éthyle ; |
| ADAMQUAT BZ 80 | solution aqueuse à 80% en poids de chlorure d'acryloxyéthyldiméthyl-benzylammonium ; |
| S-ADAMQUAT 2BZ | chlorure de l'acrylate de 1,3-bis(diméthylbenzylammonium)isopropyle ; |
| EMHQ | éther méthylique de l'hydroquinone |
| CBY1 | chlorure de benzyle |

### EXEMPLE : Synthèse du S-ADAMQUAT 2BZ par quaternisation du S-ADAME

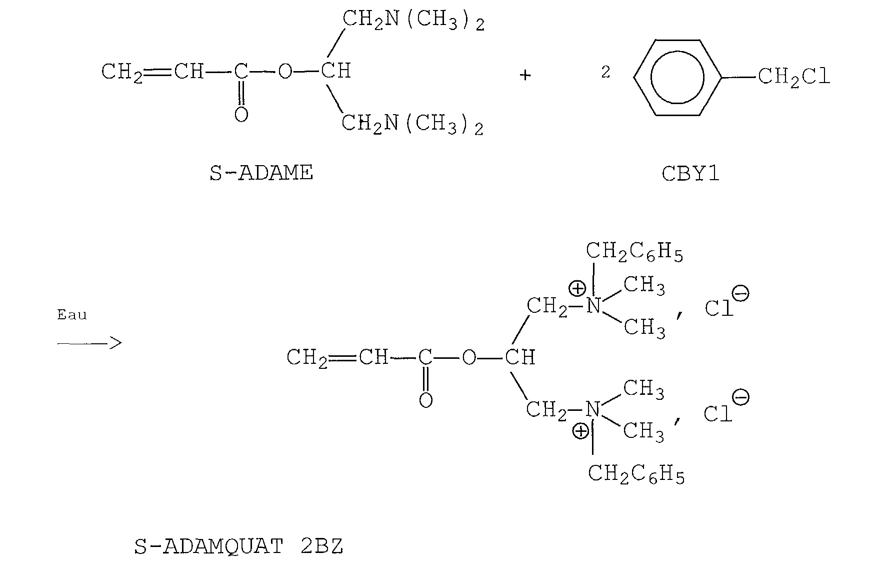

Dans un réacteur en verre de 500 ml, on charge 60 g de S-ADAME stabilisé à 800 ppm d'EMHQ et 93,2 g d'ADAMQUAT BZ 80 stabilisé à 400 ppm d'EMHQ. Le milieu sous agitation et sous bullage d'air est porté à 50°C. On ajoute alors en 3 heures 72,9 g de CBY1 (soit un débit de 24,3 g/h). Lorsque 48,6 g de CBY1 ont été introduits, ce qui correspond à 2 heures de réaction, on démarre l'ajout de 50,5 g d'eau en 4 heures (soit un débit de 12,6 g/h). Lorsque l'introduction d'eau est terminée, le milieu est maintenu sous agitation 3,5 heures supplémentaires pour convertir complètement le S-ADAME.

Après refroidissement et vidange du réacteur, on obtient 252 g de produit qui contiennent :

| | |
|---|---|
| H₂O | 19 % |
| ADAMQUAT BZ | 29 % |
| S-ADAMQUAT 2BZ | 52 % |

Le mélange ainsi obtenu est utilisé en polymérisation eau dans eau pour des applications dans le domaine des floculants.

## Revendications

1. Procédé de fabrication du composé de formule (I) : 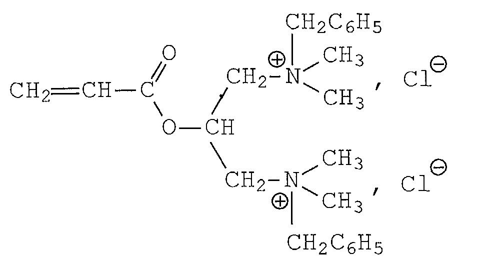 seul ou en combinaison avec au moins un monomère de formule (II) : 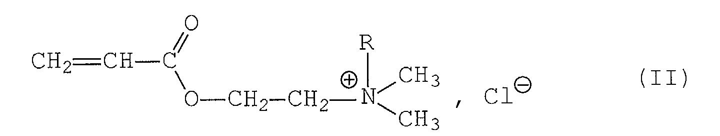 dans laquelle R représente -CH₃ ou -CH₂C₆H₅,
**caractérisé par le fait que** l'on introduit l'agent quaternisant chlorure de benzyle dans une solution, dans un solvant choisi parmi les composés des formules (I) et (II) et leurs mélanges, du composé de formule (III) : 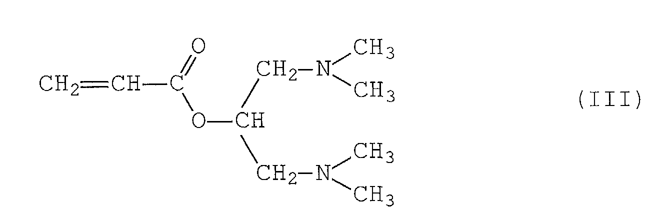 à une température de 35 à 60°C, puis qu'on ajoute de l'eau et on laisse se dérouler la réaction jusqu'à conversion complète ou sensiblement complète du composé (III), qu'on sépare une solution aqueuse de composé recherché (I) le cas échéant avec au moins un composé (II) si on a utilisé celui-ci ou ceux-ci comme solvant, et qu'on élimine l'eau le cas échéant.

2. Procédé selon la revendication 1, **caractérisé par le fait que** l'on conduit la réaction à une température de 40 à 50°C.

3. Procédé selon l'une des revendications 1 et 2, **caractérisé par le fait que** l'on conduit la réaction avec un rapport molaire composé (III)/chlorure de benzyle compris entre 1,6 et 2,0.

4. Procédé selon la revendication 3, **caractérisé par le fait que** l'on conduit la réaction avec un rapport molaire composé (III)/chlorure de benzyle compris entre 1,7 et 1,9.

5. Procédé selon l'une des revendications 1 à 4, **caractérisé par le fait que** l'on introduit le chlorure de benzyle dans la solution du composé (III) en l'espace de 8-16 heures, que l'on introduit l'eau dans la solution du composé (III) en l'espace de 2-6 heures, et que l'on commence l'introduction d'eau lorsque 10 à 80 % du chlorure de benzyle ont été introduits.

6. Procédé selon l'une des revendications 1 à 5, **caractérisé par le fait qu'**il est conduit en présence d'au moins un stabilisant choisi parmi l'éther méthylique de l'hydroquinone, l'hydroquinone, le 3,5-ditert.-butyl-4-hydroxytoluène et leurs mélanges, à raison notamment de 200 à 2000 ppm par rapport à la solution aqueuse finale et/ou en présence d'au moins un séquestrant de métaux, à raison notamment de 10 à 100 ppm par rapport à la solution aqueuse finale.

7. Procédé selon l'une des revendications 1 à 6, **caractérisé par le fait qu'**il conduit à une solution aqueuse de composé (I) uniquement, le composé (I) ayant une concentration de 50 à 75 % en poids.

8. Procédé selon l'une des revendications 1 à 6, **caractérisé par le fait qu'**il conduit à une solution aqueuse de composés (I) et (II), la solution aqueuse ayant la composition suivante, pour 100 parties en poids :
- composé (I) : 10 à 70 % en poids
- composé(s) (II): 10 à 70 % en poids
- eau : 20 à 80 % en poids

9. Homopolymères ou copolymères comportant des motifs du monomère (I) et le cas échéant d'au moins un monomère (II), ce ou ces monomères ayant été obtenus par le procédé tel que défini à l'une des revendications 1 à 8.
